# EUROPÄISCHE PATENTSCHRIFT

(11) **EP 1 296 962 B1**
(45) Veröffentlichungstag und Bekanntmachung des Hinweises auf die Patenterteilung: **05.09.2007**
(21) Anmeldenummer: 01960270.5
(22) Anmeldetag: 05.06.2001
(51) Int. Cl.: C07D 249/14

(54) **VERFAHREN ZUR HERSTELLUNG VON SUBSTITUIERTEN 5-AMINO-N-PHENYL-1,2,4-TRIAZOL-3-SULFONAMIDEN**
METHOD FOR PREPARING SUBSTITUTED 5-AMINO-N-PHENYL-1,2,4-TRIAZOLE-3-SULFONAMIDES
PROCEDE POUR PREPARER DES 5-AMINO-N-PHENYL-1,2,4-TRIAZOL-3-SULFONAMIDES SUBSTITUES

(30) Priorität: 16.06.2000 DE 10029878; 04.08.2000 DE 10038020
(43) Veröffentlichungstag der Anmeldung: 02.04.2003
(73) Patentinhaber: Bayer CropScience AG, 40789 Monheim (DE)
(72) Erfinder: BEIER, Christian, 42115 Wuppertal (DE); LANTZSCH, Reinhard, 42115 Wuppertal (DE)
(74) Vertreter: Schwenk, Norbert
(86) Internationale Anmeldenummer: PCT/EP2001/006386
(87) Internationale Veröffentlichungsnummer: WO 2001/096316

(56) Entgegenhaltungen:
- EP-A- 0 246 749
- EP-A- 0 375 061
- US-A- 4 937 350
- US-A- 4 988 812
- SHANKAR R B ET AL: "SYNTHESIS OF 1,2,4-TRIAZOLO1,5-APYRIMIDINE-2-SULFONAMID ES" JOURNAL OF HETEROCYCLIC CHEMISTRY, HETERO CORP., TAMPA, FL, US, Bd. 30, Nr. 1, Januar 1993 (1993-01), Seiten 169-172, XP000943000 ISSN: 0022-152X in der Anmeldung erwähnt

## Beschreibung

Die Erfindung betrifft ein neues Verfahren zur Herstellung von 5-Amino-N-phenyl-1,2,4-triazol-3-sulfonamiden, welche als Zwischenprodukte für Wirkstoffe in der Landwirtschaft, insbesondere für substituierte, herbizid aktive 1,2,4-Triazolo[1,5-a]pyrimidin-2-sulfonamide, bekannt sind.

Es ist bekannt, dass Triazolpyrimidinsulfonamide durch Umsetzung von Triazolpyrimidinsulfochloriden mit Alkylaminen bzw. Anilin hergestellt werden können (GB-A 951 652, EP-A 0 142 152, DE-A 36 40 155, US 4,740,233). Bei diesen Verfahren muss jedoch das Amin zum Teil erst durch Metallierung mit n-Butyllithium oder Alkalimetallhydriden aktiviert werden. Des weiteren wurde beschrieben, dass Triazolpyrimidinsulfonamide mit einem für die herbizide Wirkung gewünschten Substitutionsmuster durch eine mehrstufige Reaktion aus anderen Triazolpyrimidinsulfonamiden unter oxidativer Spaltung des Pyrimidinringes und anschließendem erneuten Ringschluss (US 4,734,123; J. Heterocyclic Chem., 30, 169 (1993)) hergestellt werden können oder aus anderen Triazolpyrimidinsulfonamiden in einer mehrstufigen Reaktion durch zwischenzeitige Überführung in Triallcylsilylderivate erhalten werden können (US 4,910,306). Die Verwendung von Organometallverbindungen sowie Verluste bei der Einführung und Abspaltung von Schutzgruppen und bei den Transformationen wirken sich bei diesen Herstellungswegen für die herbizid aktiven Triazolpyrimidinsulfonamide nachteilig aus.

Die Herstellung der Triazolpyrimidinsulfonamide auf einem anderen Weg bei höherer Ausbeute und Effizienz ist daher wünschenswert.

Dazu wurde vorgeschlagen, die Triazolpyrimidinsulfonamide aus 5-Amino-1,2,4-triazol-3-sulfonamiden herzustellen, die zuvor durch Reaktion eines entsprechenden Chlorsulfonyltriazols mit einem substituierten Anilinderivat erhalten wurden (EP-A 0 375 061). Dabei zeigte sich jedoch, dass die Herstellung der Verbindung 5-Amino-N-(2,6-dichlor-3-methylphenyl)-1,2,4-triazol-3-sulfonamid aus der Umsetzung von 2,6-Dichlor-3-methylanilin mit 5-Amino-3-chlorsulfonyl-1,2,4-triazol in Gegenwart eines organischen Lösungsmittels bei einer extrem langen Reaktionszeit von 5 Tagen lediglich zu einer Ausbeute von 67 % führt. Ebenso ergibt derselbe Reaktionsschritt bei Verwendung von Essigsäure als Lösungsmittel nach 5,5 Stunden Reaktionszeit auch nur 67 % Ausbeute.

Weitere bekannte Verfahren zur Herstellung der substituierten 5-Axniuo-N-phenyl-1,2,4-triazol-3-triazolsulfonamide, wie US 4,988,812, sind ebenfalls nicht völlig befriedigend; u.a. hinsichtlich der erzielbaren Ausbeuten (EP-A 0 246 749).

Erfindungsgemäß wurde nun gefunden, dass die für die Herstellung der Triazolpyrimidinsulfonamide benötigten substituierten 5-Amino-1,2,4-triazol-3-sulfonamide durch Umsetzung der entsprechenden reaktionsträgen, dihalogenierten Anilinderivate mit den entsprechenden Chlorsulfonyltriazolderivaten überraschenderweise in sehr guten Ausbeuten ohne nennenswerte Bildung von Nebenprodukten gelingt, wenn die Reaktion als Anilinschmelze auch ohne Zusatz einer Hilfsbase bei Temperaturen von 90°C und darüber und in Abwesenheit eines Lösungsmittels bei einer kurzen Reaktionszeit von 30 Minuten bis 6 Stunden durchgeführt wird. Das Reaktionsprodukt wie auch das unumgesetzte Anilin können bei dem erfindungsgemäßen Verfahren besonders gut in hoher Reinheit isoliert werden.

Es wurde demnach gefunden, dass man substituierte 5-Amino-N-phenyl-1,2,4-triazol-3-sulfonamide der allgemeinen Formel (I) 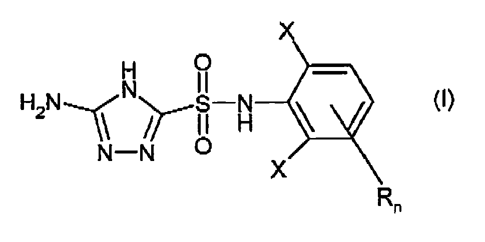 in welcher
- X: für Halogen, Cyano oder Nitro steht,
- R: für Wasserstoff oder gegebenenfalls durch Halogen substituiertes C₁-C₄-Alkyl steht, und
- n: für 1 oder 2 steht,
in sehr guten Ausbeuten und in hoher Reinheit erhält,
wenn man 5-Amino-3-chlorsulfonyl-1,2,4-triazol der Formel (II) 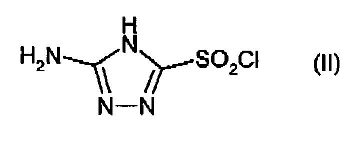 mit einem substituierten Anilin der allgemeinen Formel (III) 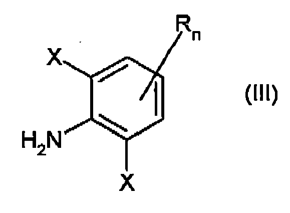 in welcher
X, n und R die oben angegebenen Bedeutungen haben,
bei Temperaturen zwischen 90°C und 150°C,
in Abwesenheit eines Lösungsmittels umsetzt.

In der Formel (I) steht X vorzugsweise für Fluor, Chlor oder Brom.

Das erfindungsgemäße Verfahren eignet sich besonders vorteilhaft zur Herstellung von 5-Amino-N-(2,6-dichlor-3-methylphenyl)-1,2,4-triazol-3-sulfonamid der Formel (Ia)

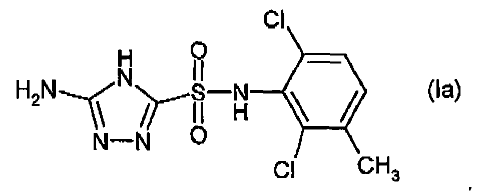

In diesem Fall wird das Produkt durch Umsetzung von 5-Amino-3-chlorsulfonyl-1,2,4-triazol der Formel (II) 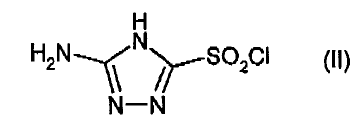 mit 2,6-Dichlor-3-methylanilin der Formel (IIIa) 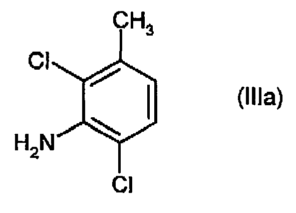 in Abwesenheit eines Lösungsmittels erhalten.

Bei dem erfindungsgemäßen Verfahren ist im Allgemeinen auch kein Hilfsbasenzusatz (z.B. Kaliumcarbonat) notwendig.

Durch die direkte Reaktion in der Schmelze und den Verzicht auf das in den Ausführungsbeispielen der EP-A 0 375 061 beschriebene Lösungsmittel und eine Reaktionsführung bei deutlich höheren Temperaturen (von 90°C und darüber) als in US 4,988,812 beschrieben, wurde überraschenderweise ein neues Verfahren gefunden, welches die Herstellung von 5-Amino-N-phenyl-1,2,4-triazol-3-sulfonamiden in sehr guten Ausbeuten und in hoher Reinheit bei kurzer Reaktionszeit erlaubt.

Durch die Reaktionsführung des erfindungsgemäßen Verfahrens in Abwesenheit eines Lösungsmittels und bei Temperaturen von 90°C und darüber wird als weiterer Vorteil die sonst erforderliche, aufwendige Wiedergewinnung des organischen Lösungsmittels nach Durchführung der Reaktion vermieden. Da auch keine Hilfsbase benötigt wird, entfällt zudem die Entstehung von Abfallprodukten. Ein wesentlicher Fortschritt des erfindungsgemäßen Verfahrens liegt in der kurzen Reaktionszeit. Schon nach 30 Minuten bis 6 Stunden werden annähernd quantitative Ausbeuten erzielt

Schließlich zeichnet sich das erfindungsgemäße Verfahren dadurch aus, dass als Nebenprodukt nur gasförmiges HCI entsteht, welches leicht zu binden ist. Das überschüssige Anilin ist durch Extraktion einfach abzutrennen und damit gut wiederzugewinnen.

Eine bevorzugte Ausführungsform des erfindungsgemäßen Verfahrens stellt daher die Variante dar, bei der nach der Umsetzung der Ausgangsstoffe in einem weiteren Reaktionsschritt das überschüssige Anilin durch Extraktion mit einem mit Wasser nicht mischbaren, organischen Lösungsmittel extrahiert und anschließend isoliert wird. Dadurch wird in einfacher Weise eine quantitative Wiedergewinnung überschüssigen Anilins in hoher Reinheit erreicht.

In einer weiteren bevorzugten Ausführungsform wird das Reaktionsprodukt der Umsetzung durch Ausfällen aus einer wässrigen Lösung unter Verwendung einer salzbildenden Säure, bevorzugt HCI, und anschließende Filtration isoliert.

Das erfindungsgemäße Verfahren stellt somit eine Bereicherung des Standes der Technik dar, da es eine sehr vorteilhafte Herstellung von 5-Amino N-phenyl-1,2,4-triazol-3-sulfonamiden erlaubt. Damit wird der Zugang zu den auf diesen Zwischenprodukten basierenden herbiziden 1,2,4-Triazolo[1,5-a]pyrimidin-2-sulfonamiden erleichtert.

Das beim erfindungsgemäßen Verfahren als Ausgangsverbindung zu verwendende 5-Amino-3-chlorsulfonyl-1,2,4-triazol der Formel (II) sowie die substituierten Aniline der Formel (III) und das 2,6-Dichlor-3-methylanilin der Formel (IIIa) sind bereits bekannt und können nach bekanten Verfahren hergestellt werden (vgl. z.B. EP-A 0 375 061).

Bei der erfindungsgemäßen Umsetzung der substituierten Aniline mit 5-Amino-3-chlorsulfonyl-1,2,4-triazol arbeitet man im Allgemeinen bei Temperaturen zwischen 90°C und 150°C, vorzugsweise zwischen 115°C und 145°C.

Bei der erfindungsgemäßen Umsetzung der substituierten Aniline mit 5-Amino-3-chlorsulfonyl-1,2,4-triazol betragen die Reaktionszeiten im Allgemeinen zwischen 30 Minuten und 6 Stunden, vorzugsweise zwischen 30 und 120 Minuten.

Zur Durchführung des erfindungsgemäßen Verfahrens setzt man zur Herstellung der Verbindungen der Formel (I) bevorzugt das Anilin im Überschuss ein. Im Allgemeinen werden je Mol 5-Amino-3-chlorsulfonyl-1,2,4-triazol der Formel (II) zwischen 1,2 und 10 Mol, vorzugsweise zwischen 1,5 und 5 Mol, des Anilins der Formel (III) eingesetzt.

Bei der bereits oben beschriebenen, bevorzugten Ausführungsform des erfindungsgemäßen Verfahrens wird die Reaktionsmischung nach der durchgeführten Umsetzung mit einer wässrigen basischen Lösung (bevorzugt mit Natronlauge) versetzt und mit einem mit Wasser praktisch nicht mischbaren organischen Lösungsmittel, wie z.B. Dichlormethan oder Toluol geschüttelt, die organische Phase mit Wasser gewaschen und getrocknet, und das als Ausgangsstoff eingesetzte, nicht umgesetzte Anilin der Formel (III) nach Verdampfung dieses Lösungsmittels isoliert.

Die Aufarbeitung und Isolierung der Verbindungen der Formel (I) kann auf übliche Weise erfolgen. Bevorzugt wird die Reaktionsmischung nach der durchgeführten Umsetzung mit einer wässrigen basischen Lösung (bevorzugt mit Natronlauge) versetzt und mit einem mit Wasser praktisch nicht mischbaren organischen Lösungsmittel, wie z.B. Dichlormethan geschüttelt, wobei eine wässrige Phase mit dem Produkt erhalten wird. Aus der wässrigen Phase kann das Endprodukt der Formel (I) nach Ansäuern mit einem mit Wasser praktisch nicht mischbaren organischen Lösungsmittel, wie z.B. Essigsäureethylester ausgeschüttelt und nach Abdestillieren des Lösungsmittels isoliert werden. Bevorzugt wird die erhaltene wässrige Phase jedoch mit einer salzbildenden Säure (z.B. HCl) versetzt. Das dabei ausfallende Endprodukt der Formel (I) kann in diesem Fall nach Filtration als Rückstand erhalten und gegebenenfalls auf übliche Weise weiter gereinigt werden.

Bei dieser bevorzugten Ausführungsform des Verfahrens wird als Fällungsreagenz bevorzugt eine Salzsäurelösung (Konzentration: einnormal bis konzentriert) verwendet und die Fällung im sauren Bereich bei pH 0 bis 6, vorzugsweise bei pH 0-3, durchgeführt.

Bei einer weiteren erfindungsgemäßen Variante des Verfahrens wird die Verbindung 5-Amino-3-chlorsulfonyl-1,2,4-triazol der Formel (II) portionsweise oder kontinuierlich dem Anilin der Formel (III) zugesetzt.

Bei dieser bevorzugten Variante erfolgt die portionsweise bzw. kontinuierliche Zugabe des Triazols zum Anilin über einen Zeitraum von mindestens 10 Minuten, bevorzugt mindestens 30 Minuten. Eine Zugabe über den Zeitraum von 10 Minuten bis 2 Stunden wird als besonders günstig angesehen.

Das erfindungsgemäße Verfahren kann auch so ausgeführt werden, dass die Umsetzung des Triazols der Formel (II) mit dem Anilin voll kontinuierlich erfolgt, d.h. dass die Edukte kontinuierlich dem Reaktionsraum zugeführt werden und das Umsetzungsprodukt kontinuierlich abgetrennt wird. Dabei wird das Anilin bevorzugt im Überschuss eingesetzt. Als Reaktionsraum eignet sich dabei insbesondere ein Rohr- oder Schleifenreaktor.

### Herstellungsbeispiele:

### Beispiel 1

In einem 250 ml Dreihalskolben mit Innenthermometer, Rückflusskühler und mechanischem Rührer werden unter Argon 66 g (0,375 mol) 2,6-Dichlor-3-methylanilin durch ein auf 130°C vorgeheiztes Ölbad auf ca. 120-130°C Innentemperatur erwärmt. Zu der gerührten Schmelze werden 27,3 g (0,15 mol) 5-Amino-3-chlorsulfonyl-1,2,4-triazol zugegeben und die Mischung kräftig gerührt. Nach 30 Minuten lässt sich per HPLC kein Sulfochlorid mehr, sondern nur noch 5-Amino-N-(2,6-dichlor-3-methylphenyl)-1,2,4-triazol-3-sulfonamid sowie 2,6-Dichlor-3-methylanilin detektieren. Man kühlt ab und versetzt das Reaktionsgemisch unter Rühren langsam mit 45 %iger Natronlauge bis auf ca. pH 12. Die alkalische Lösung wird 1 Stunde gerührt und dann 3 mal mit je 200 ml Dichlormethan extrahiert, um überschüssiges Anilin zu entfernen. Die abgetrennten, vereinigten organischen Phasen werden mit Natriumsulfat getrocknet, filtriert und das Lösungsmittel im Vakuum entfernt. Es werden 43,8 g 2,6-Dichlor-3-methylanilin (Reinheit > 99 %, HPLC, UV-Flächenprozent) erhalten. Die extrahierte wässrige Phase wird mit 32 %iger HCl bis auf ca. pH 1 angesäuert und 1 Stunde gerührt. Der ausgefallene Feststoff wird abgesaugt, mit kaltem Wasser gewaschen und über Nacht im Vakuum getrocknet. Es werden 40,0 g 5-Amino-N-(2,6-dichlor-3-methylphenyl)-1,2,4-triazol-3-sulfonamid in einer Reinheit von 98,7 % (HPLC, UV-Flächenprozent) als farbloser Feststoff mit einem Schmelzpunkt von 240-241°C erhalten. Die theoretische Ausbeute ergibt sich zu 82,7 %, bezogen auf nicht wiedergewonnenes Anilin beträgt die Ausbeute 98,4 % der Theorie.

### Beispiel 2

4,4 g (0,025 mol) 2,6-Dichlor-3-methylanilin werden auf 110°C erwärmt, 1,8 g (0,01 mol) 5-Amino-3-chlorsulfonyl-1,2,4-triazol unter Argon zugesetzt und die Suspension 90 Minuten bei dieser Temperatur gerührt. Nach Aufarbeitung analog Beispiel 1 werden 3,1 g (Reinheit 99,4 %) 2,6-Dichlor-3-methylanilin und 1,81 g (Reinheit 95,3 %) 5-Amino-N-(2,6-dichlor-3-methylphenyl)-1,2,4-triazol-3-sulfonamid erhalten. Die theoretische Ausbeute ergibt sich zu 56,2 %, bezogen auf nicht wiedergewonnenes Anilin beträgt die Ausbeute 76,1 % der Theorie.

### Beispiel 3

4,4 g (0,025 mol) 2,6-Dichlor-3-methylanilin werden auf 110°C erwärmt, 1,8 g (0,01 mol) 5-Amino-3-chlorsulfonyl-1,2,4-triazol unter Argon zugesetzt und die Suspension 6 Stunden bei dieser Temperatur gerührt. Nach Aufarbeitung analog Beispiel 1 werden 2,9 g (Reinheit 99,3 %) 2,6-Dichlor-3-methylanilin und 2,21 g (Reinheit 96,1 %) 5-Amino-N-(2,6-dichlor-3-methylphenyl)-1,2,4-triazol-3-sulfonamid erhalten. Die theoretische Ausbeute ergibt sich zu 68,6 %, bezogen auf nicht wiedergewonnenes Anilin beträgt die Ausbeute 80,5 % der Theorie.

### Beispiel 4 (Vergleichsbeispiel gemäß EP 0 375 061)

3,65 g (0,02 mol) 5-Amino-3-chlorsulfonyl-1,2,4-triazol und 3,5 g (0,02 mol) 2,6-Dichlor-3-methylanilin werden unter Argon in 40 ml Acetonitril suspendiert und unter Rückflusstemperatur 16 h gerührt. Nach Abkühlen wird der Niederschlag abgesaugt und getrocknet. Es werden 2,0 g (Reinheit 99,4 %) 2,6-Dichlor-3-methylanilin erhalten. Das Filtrat wird mit Wasser versetzt und 3 mal mit Dichlormethan extrahiert. Die wässrige Phase wird mit 32 %iger Salzsäure auf pH 1 angesäuert, der ausgefallene Feststoff abgesaugt und im Vakuum getrocknet. Es werden 0,775 g (Reinheit 96,9 %) 5-Amino-N-(2,6-dichlor-3-methylphenyl)-1,2,4-triazol-3-sulfonamid erhalten. Die theoretische Ausbeute ergibt sich zu 12 %, bezogen auf nicht wiedergewonnenes Anilin beträgt die Ausbeute 28 % der Theorie.

### Beispiel 5 (Vergleichsbeispiel gemäß EP 0 375 061)

0,9 g (0,005 mol) 5-Amino-3-chlorsulfonyl-1,2,4-triazol, 0,9 g (0,005 mol) 2,6-Dichlor-3-methylanilin und 0,7 g (0,005 mmol) getrocknetes Kaliumcarbonat werden unter Argon in 40 ml trockenem Acetonitril suspendiert und unter Rückflusstemperatur 16 h gerührt. Nach Abkühlen wird der Niederschlag abgesaugt und getrocknet. Es werden 0,8 g (88,9 % Reinheit 94,1%) 2,6-Dichlor-3-methylanilin erhalten. Im Filtrat konnte per HPLC nur die Sulfonsäure als Hydrolyseprodukt des eingesetzten Sulfochlorids detektiert werden.

### Beispiel 6

9,1 g (0,05 mol) 5-Amino-3-chlorsulfonyl-1,2,4-triazol (Reinheit 83,7 %) werden unter Argon portionsweise über 1 Stunde zu einer auf 120°C erwärmten und gerührten Schmelze von 22 g (0,125 mol) 2,6-Dichlor-3-methylanilin zugesetzt und die Suspension 90 Minuten bei dieser Temperatur gerührt. Nach Aufarbeitung analog Beispiel 1 werden 14,8 g (Reinheit 99,0 %) 2,6-Dichlor-3-methylanilin und 13,5 g (Reinheit 90,8 %) 5-Amino-N-(2,6-dichlor-3-methylphenyl)-1,2,4-triazol-3-sulfonamid erhalten. Die theoretische Ausbeute ergibt sich zu 90,9 %, bezogen auf nicht wiedergewonnenes Anilin beträgt die Ausbeute 98,1 % der Theorie.

## Patentansprüche

1. Verfahren zum Herstellen von substituierten 5-Amino-N-phenyl-1,2,4-triazol-3-sulfonamiden der allgemeinen Formel (I) 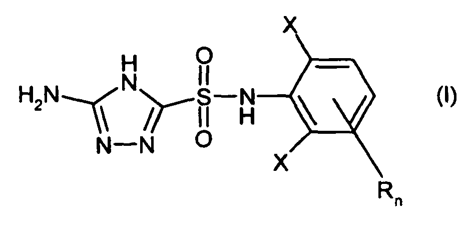 in welcher
X für Halogen, Cyano oder Nitro steht,
R für Wasserstoff oder gegebenenfalls durch Halogen substituiertes C₁-C₄-Alkyl steht, und
n für 1 oder 2 steht,
**dadurch gekennzeichnet, dass** 5-Amino-3-chlorsulfonyl-1,2,4-triazol der Formel (II) 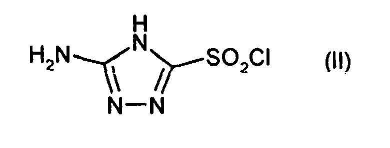 mit substituierten Anilinen der allgemeinen Formel (III) 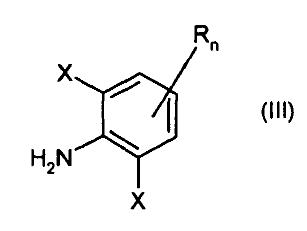 in welcher
X, n und R die oben angegebenen Bedeutungen haben,
bei Temperaturen zwischen 90°C und 150°C, in Abwesenheit eines Lösungsmittels umgesetzt wird.

2. Verfahren nach Anspruch 1, **dadurch gekennzeichnet, dass** die Verbindung der Formel (I) 5-Amino-N-(2,6-dichlor-3-methylphenyl)-1,2,4-triazol-3-sulfonamid der Formel (Ia) 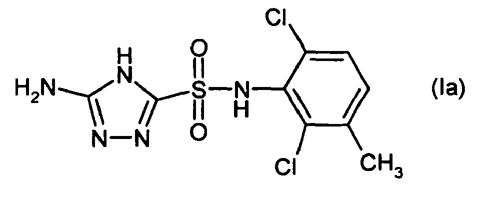 ist.

3. Verfahren nach Anspruch 1 oder 2, **dadurch gekennzeichnet, dass** die Umsetzung ohne Hilfsbasenzusatz erfolgt.

4. Verfahren nach einem der Ansprüche 1 bis 3, **dadurch gekennzeichnet, dass** die Umsetzung bei Temperaturen zwischen 115°C und 145°C durchgeführt wird.

5. Verfahren nach einem der Ansprüche 1 bis 4, **dadurch gekennzeichnet, dass** die Reaktionszeit für die Umsetzung 30 Minuten bis 6 Stunden beträgt.

6. Verfahren gemäß einem der Ansprüche 1 bis 5, **dadurch gekennzeichnet, dass** bei der Umsetzung je Mol 5-Amino-3-chlorsulfonyl-1,2,4-triazol der Formel (II) zwischen 1,2 und 10 Mol des Anilins der Formel (III) eingesetzt werden.

7. Verfahren gemäß einem der Ansprüche 1 bis 6, **dadurch gekennzeichnet, dass** nach der Umsetzung in einem weiteren Reaktionsschritt das überschüssige Anilin durch Extraktion mit einem mit Wasser nicht mischbaren, organischen Lösungsmittel extrahiert und anschließend isoliert wird.

8. Verfahren gemäß einem der Ansprüche 1 bis 7, **dadurch gekennzeichnet, dass** das Reaktionsprodukt der Umsetzung durch Ausfällen aus einer wässrigen Lösung unter Verwendung einer salzbildenden Säure und anschließende Filtration isoliert wird.

9. Verfahren gemäß einem der Ansprüche 1 bis 8, **dadurch gekennzeichnet, dass** die Verbindung 5-Amino-3-chlorsulfonyl-1,2,4-triazol der Formel (II) portionsweise oder kontinuierlich zu dem Anilin der Formel (III) zugesetzt wird.

## Claims

1. A process for preparing substituted 5-amino-N-phenyl-1,2,4-triazole-3-sulfonamides of the general formula (I) 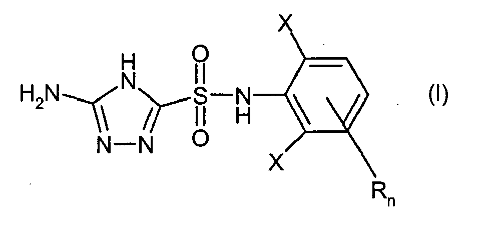 where
X is halogen, cyano or nitro,
R is hydrogen or optionally halogen-substituted C₁-C₄-alkyl, and
n is 1 or 2,
which comprises reacting 5-amino-3-chlorosulfonyl-1,2,4-triazole of the formula (II) 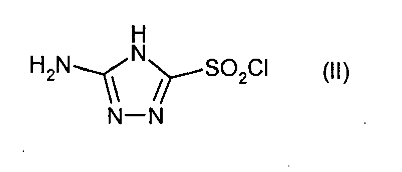 with substituted anilines of the general formula (III) 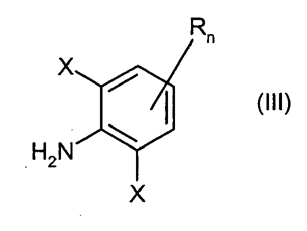 where
X, n and R are as defined above,
at temperatures of from 90°C to 150°C, in the absence of a solvent.

2. A process as claimed in claim 1, wherein the compound of the formula (I) is 5-amino-N-(2,6-dichloro-3-methylphenyl)-1,2,4-triazole-3-sulfonamide of the formula (Ia)

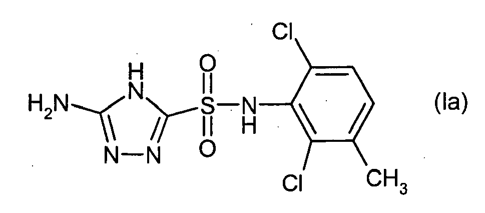

3. The process as claimed in claim 1 or 2, wherein the reaction is effected without adding an auxiliary base.

4. The process as claimed in any of claims 1 to 3, wherein the reaction is carried out at temperatures of from 115°C to 145°C.

5. The process as claimed in any of claims 1 to 4, wherein the reaction time for the reaction is from 30 minutes to 6 hours.

6. The process according to any of claims 1 to 5, wherein from 1.2 to 10 moles of the aniline of the formula (III) are used in the reaction per mole of 5-amino-3-chlorosulfonyl-1,2;4-triazole of the formula (II).

7. The process according to any of claims 1 to 6, wherein the excess aniline is extracted after the reaction in a further reaction step by extraction with a water-immiscible, organic solvent and then isolated.

8. The process as claimed in any of claims 1 to 7, wherein the reaction product of the reaction is isolated by precipitation from an aqueous solution using a salt-forming acid and subsequent filtration.

9. The process according to any of claims 1 to 8, wherein the compound 5-amino-3-chlorosulfonyl-1,2,4-triazole of the formula (11) is added in portions or continuously to the aniline of the formula (III).

## Revendications

1. Procédé de préparation de 5-amino-N-phényl-1,2,4-triazol-3-sulfonamides substitués de formule générale (I) 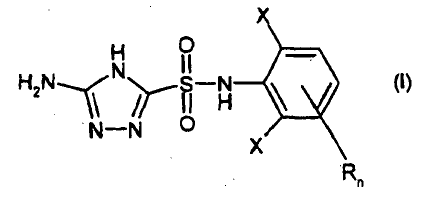 dans laquelle
X représente un halogène, cyano ou nitro,
R représente un hydrogène ou un alkyle en C₁ à C₄ éventuellement substitué par un halogène, et
n représente 1 ou 2,
**caractérisé en ce que** l'on met à réagir le 5-amino-3-chlorosulfonyl-1,2,4-triazole de formule (II) 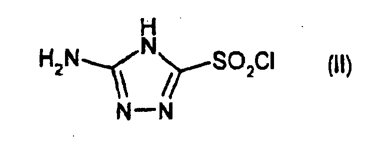 avec des anilines substituées de formule générale (III) 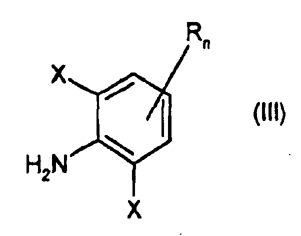 dans lesquelles
X, n et R ont les significations indiquées ci-dessus,
à des températures entre 90°C et 150 °C, en l'absence d'un solvant.

2. Procédé selon la revendication 1, **caractérisé en ce que** le composé de formule (I) est le 5-amino-N-(2,6-dichloro-3-méthylphényl)-1,2,4-triazol-3-sulfonamide de formule (Ia)

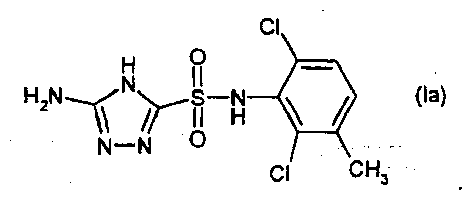

3. Procédé selon la revendication 1 ou 2, **caractérisé en ce que** l'on effectue la réaction sans ajout d'adjuvants.

4. Procédé selon l'une des revendications 1 à 3, **caractérisé en ce qu'**on effectue la réaction à des températures entre 115 °C et 145 °C.

5. Procédé selon l'une des revendications 1 à 4, **caractérisé en ce que** le temps de réaction pour la réaction est de 30 minutes à 6 heures.

6. Procédé selon l'une des revendications 1 à 5, **caractérisé en ce qu'**au cours de la réaction, par mole de 5-amino-3-chlorosulfonyl-1,2,4-triazole de formule (II) on utilise entre 1,2 et 10 mol de l'aniline de formule (III).

7. Procédé selon l'une des revendications 1 à 6, **caractérisé en ce qu'**après la réaction, dans une autre étape de réaction, on extrait l'aniline en excès par extraction avec un solvant organique, non miscible à l'eau et ensuite on l'isole.

8. Procédé selon une des revendications 1 à 7, **caractérisé en ce qu'**on isole le produit de réaction de la réaction par précipitation dans une solution aqueuse en utilisant un acide formant un sel et ensuite par filtration.

9. Procédé selon une des revendications 1 à 8, **caractérisé en ce que** l'on ajoute le composé 5-amino-3-chlorosulfonyl-1,2,4-triazole de formule (II) par portion ou en continu à l'aniline de formule (III).
